(19) Europäisches Patentamt
European Patent Office
Office européen des brevets

(11) **EP 2 562 542 B1**

(12) **EUROPÄISCHE PATENTSCHRIFT**

(45) Veröffentlichungstag und Bekanntmachung des Hinweises auf die Patenterteilung:
**28.01.2015 Patentblatt 2015/05**

(51) Int Cl.:
*G01N 33/49* $^{(2006.01)}$     *G01N 33/86* $^{(2006.01)}$

(21) Anmeldenummer: **11178236.3**

(22) Anmeldetag: **22.08.2011**

(54) **Dynamischen Bestimmung der Thrombozytenfunktion**

Dynamically determining the thrombocyte function

Détermination dynamique de la fonction des thrombocytes

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**27.02.2013 Patentblatt 2013/09**

(73) Patentinhaber: **Siemens Healthcare Diagnostics Products GmbH**
**35041 Marburg (DE)**

(72) Erfinder: **De Haan, Jacob**
**35315 HOMBERG/OHM (DE)**

(56) Entgegenhaltungen:
**EP-A1- 1 850 134     EP-A2- 1 477 807**

- **BORZINI P ET AL: "Evaluation of the hemostatic function of stored platelet concentrates using the platelet function analyzer (PFA-100TM)", HAEMATOLOGICA, FONDAZIONE FERRATA STORTI, ROME, IT, Bd. 84, Nr. 12, 1. Dezember 1999 (1999-12-01), Seiten 1104-1109, XP008082936, ISSN: 0390-6078**
- **KUNDU S K ET AL: "Description of and in vitro platelet function analyzer-PFA-100", SEMINARS IN THROMBOSIS AND HEMOSTASIS, STUTTGART, DE, Bd. 21, Nr. Suppl.2, 1. Januar 1995 (1995-01-01), Seiten 106-112, XP008082934, ISSN: 0094-6176**
- **PAUL HARRISON: "The role of PFA-100R testing in the investigation and management of haemostatic defects in children and adults", BRITISH JOURNAL OF HAEMATOLOGY, Bd. 130, Nr. 1, 1. Juli 2005 (2005-07-01), Seiten 3-10, XP55014331, ISSN: 0007-1048, DOI: 10.1111/j. 1365-2141.2005.05511.x**

**Beschreibung**

Die Erfindung liegt auf dem Gebiet der Gerinnungsdiagnostik und betrifft ein in vitro-Verfahren zur Bestimmung der Thrombozytenfunktion mit Hilfe von Messzellen. Das Verfahren ermöglicht die Gewinnung von Testergebnissen, die direkt miteinander vergleichbar sind, und somit z.B. eine Verlaufskontrolle in Patienten mit wiederholten Bestimmungen ermöglicht.

Physiologische Vorgänge, die einerseits die Fluidität des Blutes im Gefäßsystem gewährleisten und andererseits durch die Bildung von Blutgerinnseln die Vermeidung von extravasalen Blutverlusten sicherstellen, werden unter dem Begriff Hämostase zusammengefasst. An der Regulation der Hämostase sind eine Vielzahl von Proteinfaktoren beteiligt sowie auch zelluläre Komponenten, wie z.B. die Thrombozyten (Blutplättchen). Im Falle einer Gefäßverletzung kommt es zuerst zur Anlagerung von Thrombozyten an das subendotheliale Kollagen. Diese Adhäsion wird durch Adhäsivproteine, wie den von-Willebrand-Faktor (VWF) vermittelt. Während des Adhäsionsvorgangs werden die Thrombozyten aktiviert und schütten Mediatoren aus ihren Granulae aus, wodurch die Aggregation weiterer Thrombozyten sowie eine Verstärkung der Aktivierung induziert wird. Auf diese Weise erfolgt ein primärer Gefäßwandverschluss (primäre Hämostase), der erst durch weitere Reaktionen des plasmatischen Gerinnungssystems (sekundäre Hämostase) stabilisiert wird. Fehlregulationen dieser Prozesse können zu einer Thromboseneigung oder einer Blutungsneigung führen und je nach Schweregrad lebensbedrohliche Folgen haben.

In der Gerinnungsdiagnostik sind verschiedene Verfahren und Systeme bekannt, mit deren Hilfe bestimmt werden kann, ob das Blut eines Patienten einwandfrei gerinnen kann oder ob eine Gerinnungsstörung vorliegt. Im Fall einer Gerinnungsstörung ist es häufig erforderlich, präzisere Kenntnisse über die Ursache der vorliegenden Störung zu erlangen, um die optimalen therapeutischen Maßnahmen auswählen zu können. Eine wichtige Teilfunktion des Gerinnungssystems, die gezielt untersucht werden kann, ist die primäre Hämostase, die wesentlich von der Funktionsfähigkeit der Thrombozyten abhängt.

Ein bekanntes Verfahren zur Untersuchung der Thrombozytenfunktion ist die Blutungszeitbestimmung. Es handelt sich dabei um einen in vivo-Globaltest, der die primäre Hämostase erfasst. Die Blutungszeit wird bestimmt, indem dem Patienten eine kleine Schnitt- oder Stichverletzung zugefügt wird und die Zeit bis zum Blutungsstillstand gemessen wird. Es handelt sich um einen invasiven, schwer standardisierbaren, grob informativen Test, der vor allem in Notfallsituationen angewendet wird, um einen Überblick über die primäre Hämostase zu erhalten. Die Einnahme von Thrombozytenaggregationshemmern führt oft zu einer Verlängerung der Blutungszeit. Nachteilig an der Blutungszeitbestimmung ist, dass bei einer normalen Blutungszeit eine Thrombozytenfunktionsstörung nicht ausgeschlossen werden kann.

In vitro-Verfahren ermöglichen einen wesentlich empfindlicheren Nachweis von Thrombozytenfunktionsstörungen. Bei diesen Verfahren wird üblicherweise in einer Vollblutprobe oder in einer Probe von plättchenreichem Plasma (PRP) die Aggregation der Thrombozyten durch Zugabe eines Aktivators induziert und die Aggregationsreaktion gemessen. Die meistverwendeten Aktivatoren, die zur Induktion der Thrombozytenaggregation verwendet werden, sind: ADP (Adenosin-5'-diphosphat), Kollagen, Epinephrin (Adrenalin), Ristocetin und verschiedene Kombinationen davon sowie Thrombin, TRAP (Thrombin-Receptor-Activating-Protein) oder Serotonin.

Ein bekanntes System zur Bestimmung der Thrombozytenfunktion in vitro ist das sogenannte Platelet Function Analyzer System, kurz PFA-System (PFA-100®, PFA-200, Siemens Healthcare Diagnostics Products GmbH, Marburg, Deutschland). Mit Hilfe des PFA-Systems wird in Vollblutproben die primäre Hämostase unter Flussbedingungen und damit in Gegenwart von hohen Scherkräften gemessen.

Zur Simulation der Flussbedingungen und der Scherkräfte, wie sie in kleineren arteriellen Blutgefäßen herrschen, wird in einer PFA-Messzelle, die in PFA-Analysegerät eingesetzt wird, ein Unterdruck von etwa -40mbar erzeugt, und das Citratvollblut, dass sich in einem Probenreservoir befindet, strömt durch eine Kapillare, die einen Durchmesser von etwa 200 μm hat. Die Kapillare mündet in eine Messkammer, die mit einem Trennelement, z.B. einer Membran abgeschlossen ist, welche eine kapillare zentrale Öffnung (Apertur) enthält, durch die das Blut aufgrund des Unterdrucks durchtritt. In den meisten Fällen ist die Membran, zumindest in dem Bereich um die Apertur mit einem oder mehreren Aktivatoren, die die Thrombozytenaggregation induzieren, versetzt, so dass das vorbeiströmende Blut mit den aggregationsinduzierenden Substanzen im Bereich der Apertur in Kontakt kommt. Infolge der induzierten Adhäsion und Aggregation der Thrombozyten bildet sich im Bereich der Apertur ein Thrombozytenpfropf (Thrombus), der die Membranöffnung verschließt und den Blutfluss stoppt. In diesem System wird die Zeit gemessen, die bis zum Verschluss der Membranöffnung benötigt wird. Diese sogenannte Verschlusszeit korreliert mit der Funktionsfähigkeit der Thrombozyten. Eine Messzelle zur Verwendung in einem Verfahren zu Bestimmung der Thrombozytenfunktion anhand der Verschlusszeit ist z.B. in dem Patentdokument WO 97/34698 beschrieben. Bislang werden in dem Verfahren zur Bestimmung der Verschlusszeit Messzellen verwendet, die mit einer Membran ausgestattet sind, die mit Kollagen (Kol) und zusätzlich entweder mit ADP oder mit Epinephrin (Epi) beschichtet ist. Andere Messzellen, die insbesondere zur Bestimmung von Antithrombotika aus der Gruppe der P2Y(12)-Antagonisten, wie z.B. von Clopidogrel, geeignet sind, sind mit einer Membran ausgestattet, die ADP und Prostaglandin E1 enthält (EP-A1-1850134).

Ein bekanntes Verfahren zur Bestimmung der Thrombozytenfunktion in einer Probe mittels eines PFA-Systems, umfasst folgende Verfahrensschritte:

a) Verwendung einer Messzelle, wobei die Messzelle folgende folgende Elemente aufweist:

i) eine Haltekammer zum Halten der Probe,
ii) eine Kapillare, durch die die Probe von der Haltekammer in eine Messkammer geleitet wird,
iii) eine Messkammer, die durch ein Trennelement in zwei Kompartimente unterteilt ist, wobei das erste Kompartiment die Probe aus der Kapillare aufnimmt,
iv) ein Trennelement, das die Messkammer in zwei Kompartimente unterteilt und das eine Öffnung aufweist, durch welche die Probe aus dem ersten Kompartiment in das zweite Kompartiment fließen kann;

b) Befüllen der Haltekammer der Messzelle mit der Probe;

c) Platzieren der Messzelle in einem Gerät zur automatischen Bestimmung der Thrombozytenfunktion, wobei das Gerät folgende Elemente aufweist:

i) Mittel zum Anlegen eines Unterdrucks in der Messkammer der Messzelle,
ii) Mittel zur Bestimmung der Flussrate der Probe, die erzeugt wird, wenn in der Messkammer der Messzelle ein Unterdruck angelegt ist;

d) Anlegen eines Unterdrucks in der Messkammer der Messzelle und Durchleiten der Probe durch die Kapillare und durch die Öffnung des Trennelements;

e) Bestimmen der Flussrate der Probe, welche durch das Anlegen eines Unterdrucks durch die Kapillare und durch die Öffnung des Trennelements gezogen wird; und

f) Bestimmen der Verschlusszeit.

Die Messung der Flussrate der Probe, welche durch das Anlegen eines Unterdrucks aus der Messzelle gezogen wird, kann bekanntermaßen dadurch bestimmt werden, dass das Volumen gemessen wird, das das Mittel zum Anlegen eines Unterdrucks in der Messkammer der Messzelle pro Zeiteinheit aus der Messzelle zieht. Das Mittel zum Anlegen eines Unterdrucks besteht bevorzugterweise aus einem Zylinder mit einem Kolben, wobei der Kolben über einen Schrittmotor und eine Kolbenstange in axialer Richtung des Zylinders beweglich ist. Durch Bewegen des Kolbens wird ein Unterdruck in der Messzelle erzeugt. In der Messkammer der Messzelle bzw. in dem Raum, der durch Verbinden der Messzelle mit dem Zylinder geschaffen wird und in welchem der Unterdruck aufgebaut wird, misst ein Drucksensor den herrschenden Druck. In einer Steuervorrichtung wird der gemessene Druck mit einem vorgegebenen Drucksollwert verglichen. Beim Einfließen von Blut in die Kapillare steigt der Druck. Um den Druck konstant zu halten, z.B. auf -40 mbar, steuert die Steuervorrichtung den Schrittmotor, der den Kolben entsprechend axial bewegt. Über den pro Zeiteinheit zurückgelegten Weg des Kolbens bzw. die Schrittzahl in dem Zylinder mit bekanntem Durchmesser kann die Flussrate der Probe, welche aus der Messzelle gezogen wird, berechnet werden.
Die Flussrate der Probe (z.B. $\mu$L/min), d.h. das Probenvolumen, welches pro Zeiteinheit durch die Öffnung des Trennelements tritt, nimmt typischerweise über die Zeit ab, da der Thrombozytenpropf die Apertur zunehmend verengt und so den Durchtritt der Probenflüssigkeit erschwert.
Als Testergebnis wird die Verschlusszeit in Sekunden ausgegeben. Die Verschlusszeit ist definiert als der Zeitpunkt, in dem die Flussrate der Probe für eine Dauer von drei Sekunden weniger als 10 % der initialen Flussrate betragen hat. Die initiale Flussrate ist die Flussrate zu Beginn der Messung, wenn sich an der Apertur noch kein Thrombozytenpfropf gebildet hat, aber das Totvolumen bereits aus dem System gezogen wurde. Abhängig von Messzellentyp und Probenviskosität liegt die initiale Flussrate typischerweise zwischen etwa 140 und 200 $\mu$L/min.
Weicht die Verschlusszeit einer Patientenprobe vom Referenzbereich ab, weist dies auf eine Störung der Thrombozytenfunktion hin. Verlängerte Verschlusszeiten weisen auf das Vorliegen einer Thrombozytendysfunktion im Sinne einer verminderten Aggregationsfähigkeit hin. Verkürzte Verschlusszeiten weisen auf das Vorliegen einer Thrombozytendysfunktion im Sinne einer erhöhten Aggregationsfähigkeit hin.
Die Verschlusszeit einer Probe eines gesunden Spenders mit normaler Thrombozytenfunktion hängt von vielen Faktoren ab.
In erster Linie wird die Verschlusszeit beeinflusst durch die Art der verwendeten Messzelle. Wie oben erläutert, werden Messzellen verwendet, die verschiedene Kombinationen von Thrombozytenaktivatoren oder -inhibitoren enthalten. Bei Verwendung einer Messzelle (Kol/Epi), die Kollagen und Epinephrin enthält, liegt die Verschlusszeit einer Normalprobe zwischen 85-165 Sekunden. Bei Verwendung einer Messzelle (Kol/ADP), die Kollagen und ADP enthält, liegt die Verschlusszeit einer Normalprobe zwischen 71-118 Sekunden. Bei Verwendung einer Messzelle (ADP/PGE 1, INNOVANCE® PFA P2Y), die ADP und Prostaglandin E1 enthält, liegt die Verschlusszeit einer Normalprobe zwischen 51-90 Sekunden. Die starken Abweichungen der Referenzbereiche führen dazu, dass eine Verschlusszeit als solche immer nur im Zusammenhang mit dem verwendeten Messzellentyp gedeutet werden kann. Eine Verschlusszeit von beispielsweise 130 Sekunden ist bei Kol/Epi-Messzellen ein normales Ergebnis, bei Kol/ADP- oder ADP/PGE1-Messzellen hingegen ein abnormales Ergebnis, das auf eine Thrombozytenfunktionsdefizienz schließen lässt.

Typischerweise entsprechen höhere Testergebnisse (also verlängerte Verschlusszeiten) einer niedrigeren Thrombozytenfunktion, und umgekehrt. In der Praxis hat sich gezeigt, dass für einige Anwender die Interpretation von Verschlusszeitergebnissen problematisch ist, da eine verkürzte Verschlusszeit eine erhöhte Thrombozytenfunktion und eine verlängerte Verschlusszeit ein eine verminderte Thrombozytenfunktion anzeigt. Daher ist es wünschenswert, das zuvor beschriebene bekannte Verfahren zur Bestimmung der Thrombozytenfunktion so zu modifizieren, dass ein Ergebnis ausgegeben wird, das positiv mit der Thrombozytenfunktion korreliert.

Ein weiterer Nachteil der Verschlusszeitbestimmung besteht darin, dass Proben mit stark verminderter Thrombozytenfunktion zu keinem Zeitpunkt eine Flussrate zeigen, die weniger als 10 % der initialen Flussrate beträgt. Dies hat zur Folge, dass kein Aperturverschluss und keine Verschlusszeitbestimmung erfolgen kann. Üblicherweise wird dann als Testergebnis "Verschlusszeit > 300 s" angegeben. Eine feinere Differenzierung solcher Proben mit stark verminderter Thrombozytenfunktion wäre wünschenswert und würde sogar eine Verlaufskontrolle bei Patienten mit wiederholten Messungen ermöglichen.

Ein weiterer Nachteil der Verschlusszeitbestimmung besteht darin, dass die Verschlusszeiten im Vergleich zu einem Normalbereich interpretiert werden müssen. Es wäre wünschenswert bessere und einfacher interpretierbare Testergebnisse zu erhalten.

Diese Aufgaben werden erfindungsgemäß dadurch gelöst, dass zunächst eine Verschlusszeitbestimmung gemäß dem Stand der Technik durchgeführt wird und dann ein Testergebnis nach folgender Formel bestimmt wird:

$$PHC = \frac{100 - FF\ [\%\ von\ IF]}{(CT - CT_M) + 90}$$

mit
FF = finale Flussrate,
IF = initiale Flussrate,
CT = Verschlusszeit der Probe [s],
$CT_M$ = Referenzverschlusszeit [s] für normale Thrombozytenfunktion.

Das so ermittelte Testergebnis ist ein Maß für die Thrombozytenfunktion im Vergleich zur Norm. Es kann auch als Primäre Hämostase Kapazität (Primary Hemostasis Capacity, PHC) bezeichnet werden.

Bevorzugterweise wird die Thrombozytenfunktion in % der Norm bestimmt, indem der PHC-Wert mit 100 multipliziert wird.

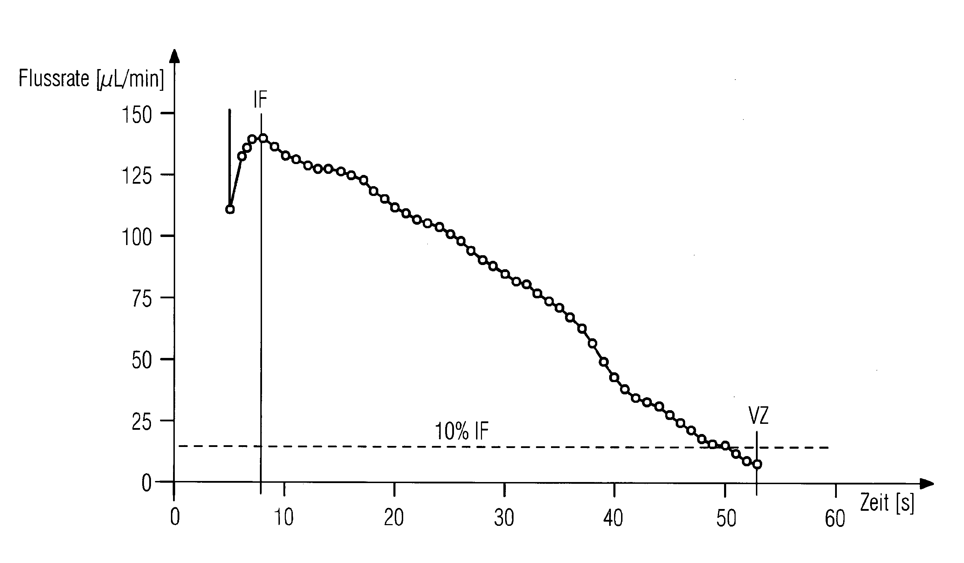

Die "initiale Flussrate" ist die maximale Flussrate der Probenflüssigkeit zu Beginn der Messung. Die Bestimmung der initialen Flussrate erfolgt bevorzugterweise dadurch, dass die Flussrate, d.h. das Volumen, das pro Zeiteinheit aus der Messzelle gezogen wird, ab dem Beginn der Messung, d.h. ab dem Zeitpunkt, in dem ein Unterdruck in der Messkammer der Messzelle angelegt wird, kontinuierlich bestimmt wird. Ganz zu Beginn der Messung kommt es unter anderem wegen des zunächst angesaugten Totvolumens zu gewissen Schwankungen in der Flussrate bis sich schließlich eine kontinuierlich abnehmende Flussrate einstellt. Die initiale Flussrate ist definiert als die Flussrate, die gemessen wurde bevor die Flussrate schließlich für mindestens 10 Sekunden kontinuierlich abnimmt (siehe auch Figur 1).

Unter dem Begriff "finale Flussrate" (FF) ist eine Flussrate zu verstehen, die zeitlich nach der initialen Flussrate bestimmt wird. Da die Flussrate der Probe (z.B. µL/min) infolge der zunehmenden Verengung der Apertur abnimmt, ist die finale Flussrate ein Bruchteil der initialen Flussrate (% von IF).

Üblicherweise ist für die finale Flussrate ein Wert von 10 % der initialen Flussrate vorbestimmt, und der Zeitpunkt, in dem die Flussrate der Probe für eine Dauer von drei Sekunden die finale Flussrate unterschreitet, wird als Verschlusszeit (CT oder VZ) bezeichnet.

Für den Fall, dass nach hinreichend langer Testdauer zum Testende, nach 300 Sekunden, keine Flussrate gemessen werden konnte, die gegenüber der initialen Flussrate um 90 % vermindert ist und dadurch keine Verschlusszeit bestimmt werden kann, wird die tatsächliche Flussrate zum Testende gemessen und als finale Flussrate bestimmt. Als Verschlusszeit wird die gesamte Testdauer, also 300 Sekunden, bestimmt.

Unter dem Begriff "Referenzverschlusszeit für normale Thrombozytenfunktion" ($CT_M$) ist eine messzellentypspezifische, mittlere Verschlusszeit in Sekunden zu verstehen. Die Referenzverschlusszeit für normale Thrombozytenfunktion wird in Vorversuchen für einen bestimmten Messzellentyp mit Normalproben von gesunden Spendern ermittelt. Die Referenzverschlusszeit für normale Thrombozytenfunktion entspricht einer Thrombozytenfunktion von 100 %.

In dem Fall, dass für eine Probe eine Verschlusszeit bestimmbar ist, wird der Zähler in der Formel zur PHC-Bestimmung immer einen Wert von 90 haben, da die finale Flussrate FF per definitionem den Wert 10 [% von IF] beträgt.

Der PHC-Wert, also das Maß für die Thrombozytenfunktion, hängt dann ausschließlich von der bestimmten Verschlusszeit (CT) ab. Ist diese infolge einer verminderten Thrombozytenaktivität größer als die Referenzverschlusszeit für normale Thrombozytenfunktion, dann hat der Nenner in der Formel zur PHC-Bestimmung einen Wert >90, und der PHC-Wert ist <1 bzw. <100 %. PHC-Werte, die kleiner als 1 bzw. als 100 % sind, zeigen also eine verminderte Thrombozytenfunktion an. Ist die bestimmte Verschlusszeit infolge einer erhöhten Thrombozytenaktivität jedoch größer als die Referenzverschlusszeit für normale Thrombozytenfunktion, dann hat der Nenner in der Formel zur PHC-Bestimmung einen Wert <90, und der PHC-Wert ist >1 bzw. >100 %. PHC-Werte, die größer als 1 bzw. als 100 % sind, zeigen also eine erhöhte Thrombozytenfunktion an.

In dem Fall, dass für eine Probe keine Verschlusszeit bestimmbar ist, weil während der gesamten Testdauer keine Verminderung der Flussrate um 90 % gegenüber der initialen Flussrate erzielt wird, wird die Gesamttestdauer als Verschlusszeit bestimmt, also 300 Sekunden, und die tatsächliche Flussrate, die zu diesem Zeitpunkt gemessen wurde, wird als finale Flussrate bestimmt (FF). In solchen Fällen hängt der PHC-Wert, also das Maß für die Thrombozytenfunktion, dann ausschließlich von der am Ende der Testzeit bestimmten tatsächlichen Flussrate ab.

## FIGURENBESCHREIBUNG

### FIGUR 1

Figur 1 zeigt eine typische Flusskurve einer Vollblutprobe eines gesunden Spenders mit normaler Thrombozytenaktivität in einer INNOVANCE® PFA P2Y-Messzelle. Nach anfänglichen Schwankungen der Flussrate zu Beginn der Messung stellt sich eine kontinuierlich abnehmende Flussrate ein. Die Flussrate, die am Beginn der kontinuierlichen Abnahme gemessen wurde, stellt die initiale Flussrate (IF) dar. Die Verschlusszeit (VZ) ist definiert als der Zeitpunkt, in dem die Flussrate für eine Dauer von drei Sekunden weniger als 10 % der initialen Flussrate betragen hat.

## BEISPIELE

### Beispiel 1: Erfindungsgemäße Bestimmung der Thrombozytenfunktion in einem PFA-100 System

Es wurden Vollblutproben (3,8 % Zitratvollblut) von 108 gesunden Spendern vor und nach der Einnahme von 300 mg Acetylsalicylsäure (ASS) entnommen. Die Thrombozytenfunktion wurde mit Hilfe des erfindungsgemäßen Verfahrens und unter Verwendung von Messzellen, die mit einer Membran ausgestattet waren, die mit Kollagen (Kol) und mit Epinephrin (Epi) beschichtet war (Kol/Epi), in einem PFA-100® System (Siemens Healthcare Diagnostics Products GmbH) bestimmt.

Die Referenzverschlusszeit für normale Thrombozytenfunktion ($CT_M$) für die Kol/Epi-Messzelle wurde bestimmt, indem aus den Kol/Epi-Verschlusszeiten der 108 vorgenannten gesunden Spender vor der Einnahme von ASS der Mittelwert gebildet wurde. Die kalkulierte Referenzverschlusszeit für normale Thrombozytenfunktion ($CT_M$) für Kol/Epi-Messzellen betrug 121 Sekunden.

In Tabelle 1 sind die Ergebnisse der Verschlusszeit- und PHC-Bestimmung für die 108 verschiedenen Spender vor und nach der Einnahme von ASS zusammengefasst.

Es zeigt sich, dass Proben, bei denen aufgrund einer stark verminderten Thrombozytenfunktion keine Verschlusszeitbestimmung durchgeführt werden konnte und die deshalb eine Verschlusszeit von 300 s als Verschlusszeitergebnis erhalten (siehe z.B. Spender Nr. 3, 6, 8-12 nach der Einnahme von ASS), mit Hilfe des erfindungsgemäßen Verfahrens feiner differenziert werden können. Die Proben der Spender Nr. 3, 6, 8-12 (nach der Einnahme von ASS), zum Beispiel, weisen PHC-Werte von 11 % bis 32 % auf.

Tabelle 1 Verschlusszeiten und PHC-Werte mit Kol/Epi-Messzellen vor und nach der Einnahme von ASS

| Spender | Vor der Einnahme von 300 mg ASS | | Nach der Einnahme von 300 mg ASS | |
| :---: | :---: | :---: | :---: | :---: |
| | CT [s] | PHC [%] | CT [s] | PHC [%] |
| 1 | 75 | 205% | 125 | 96% |
| 2 | 77 | 196% | 178 | 61% |
| 3 | 112 | 111% | 300 | 19% |

(fortgesetzt)

| Spender | Vor der Einnahme von 300 mg ASS | | Nach der Einnahme von 300 mg ASS | |
|---|---|---|---|---|
| | CT [s] | PHC [%] | CT [s] | PHC [%] |
| 4 | 119 | 102% | 207 | 51% |
| 5 | 105 | 122% | 144 | 80% |
| 6 | 156 | 72% | 300 | 11% |
| 7 | 101 | 129% | 147 | 78% |
| 8 | 129 | 92% | 300 | 22% |
| 9 | 98 | 134% | 300 | 32% |
| 10 | 102 | 127% | 300 | 30% |
| 11 | 127 | 94% | 300 | 24% |
| 12 | 128 | 93% | 300 | 26% |
| 13 | 104 | 123% | 140 | 83% |
| 14 | 108 | 117% | 98 | 134% |
| 15 | 95 | 141% | 135 | 87% |
| 16 | 117 | 105% | 300 | 22% |
| 17 | 119 | 102% | 300 | 21% |
| 18 | 135 | 87% | 300 | 13% |
| 19 | 166 | 67% | 262 | 39% |
| 20 | 133 | 88% | 300 | 17% |
| 21 | 129 | 92% | 258 | 40% |
| 22 | 121 | 100% | 168 | 66% |
| 23 | 90 | 153% | 162 | 69% |
| 24 | 138 | 84% | 193 | 56% |
| 25 | 92 | 148% | 130 | 91% |
| 26 | 111 | 113% | 163 | 68% |
| 27 | 130 | 91% | 199 | 54% |
| 28 | 138 | 84% | 280 | 36% |
| 29 | 120 | 101% | 178 | 61% |
| 30 | 110 | 114% | 176 | 62% |
| 31 | 106 | 120% | 185 | 58% |
| 32 | 158 | 71% | 300 | 13% |
| 33 | 154 | 73% | 300 | 10% |
| 34 | 159 | 70% | 300 | 13% |
| 35 | 115 | 107% | 300 | 17% |
| 36 | 114 | 108% | 300 | 22% |
| 37 | 104 | 123% | 300 | 21% |
| 38 | 119 | 102% | 300 | 25% |
| 39 | 125 | 96% | 300 | 16% |
| 40 | 112 | 111% | 300 | 23% |

(fortgesetzt)

| Spender | Vor der Einnahme von 300 mg ASS | | Nach der Einnahme von 300 mg ASS | |
|---|---|---|---|---|
| | CT [s] | PHC [%] | CT [s] | PHC [%] |
| 41 | 111 | 113% | 168 | 66% |
| 42 | 105 | 122% | 157 | 71% |
| 43 | 116 | 106% | 300 | 25% |
| 44 | 113 | 110% | 300 | 28% |
| 45 | 118 | 103% | 185 | 58% |
| 46 | 90 | 153% | 300 | 33% |
| 47 | 114 | 108% | 300 | 21% |
| 48 | 117 | 105% | 160 | 70% |
| 49 | 110 | 114% | 109 | 115% |
| 50 | 95 | 141% | 105 | 122% |
| 51 | 112 | 111% | 300 | 18% |
| 52 | 96 | 138% | 84 | 170% |
| 53 | 133 | 88% | 300 | 10% |
| 54 | 97 | 136% | 98 | 134% |
| 55 | 101 | 129% | 241 | 43% |
| 56 | 107 | 118% | 98 | 134% |
| 57 | 104 | 123% | 196 | 55% |
| 58 | 162 | 69% | 300 | 6% |
| 59 | 90 | 153% | 184 | 59% |
| 60 | 120 | 101% | 139 | 83% |
| 61 | 115 | 107% | 300 | 21% |
| 62 | 121 | 100% | 300 | 17% |
| 63 | 125 | 96% | 114 | 108% |
| 64 | 106 | 120% | 300 | 25% |
| 65 | 118 | 103% | 158 | 71% |
| 66 | 117 | 105% | 300 | 21% |
| 67 | 161 | 69% | 300 | 4% |
| 68 | 178 | 61% | 300 | 10% |
| 69 | 136 | 86% | 300 | 13% |
| 70 | 193 | 56% | 152 | 74% |
| 71 | 93 | 145% | 131 | 90% |
| 72 | 103 | 125% | 300 | 14% |
| 73 | 102 | 127% | 183 | 59% |
| 74 | 155 | 73% | 300 | 9% |
| 75 | 113 | 110% | 300 | 22% |
| 76 | 110 | 114% | 300 | 26% |
| 77 | 130 | 91% | 300 | 17% |

(fortgesetzt)

| Spender | Vor der Einnahme von 300 mg ASS | | Nach der Einnahme von 300 mg ASS | |
|---|---|---|---|---|
| | CT [s] | PHC [%] | CT [s] | PHC [%] |
| 78 | 118 | 103% | 161 | 69% |
| 79 | 187 | 58% | 300 | 7% |
| 80 | 106 | 120% | 144 | 80% |
| 81 | 113 | 110% | 300 | 25% |
| 82 | 145 | 79% | 300 | 13% |
| 83 | 146 | 78% | 151 | 75% |
| 84 | 139 | 83% | 231 | 45% |
| 85 | 150 | 76% | 300 | 13% |
| 86 | 104 | 123% | 189 | 57% |
| $7 | 124 | 97% | 236 | 44% |
| 88 | 101 | 129% | 300 | 29% |
| 89 | 135 | 87% | 175 | 63% |
| 90 | 148 | 77% | 291 | 35% |
| 91 | 106 | 120% | 150 | 76% |
| 92 | 145 | 79% | 300 | 9% |
| 93 | 93 | 145% | 300 | 29% |
| 94 | 184 | 59% | 109 | 115% |
| 95 | 126 | 95% | 300 | 17% |
| 96 | 101 | 129% | 300 | 21% |
| 97 | 107 | 118% | 300 | 20% |
| 98 | 109 | 115% | 300 | 26% |
| 99 | 87 | 161% | 300 | 32% |
| 100 | 124 | 97% | 130 | 91% |
| 101 | 134 | 87% | 300 | 19% |
| 102 | 152 | 74% | 300 | 15% |
| 103 | 121 | 100% | 300 | 17% |
| 104 | 138 | 84% | 300 | 9% |
| 105 | 144 | 80% | 300 | 11% |
| 106 | 106 | 120% | 300 | 22% |
| 107 | 111 | 113% | 196 | 55% |
| 108 | 135 | 87% | 151 | 75% |
| Mittel | 121 | 106% | 237 | 45% |

**Patentansprüche**

1. Verfahren zur Bestimmung der Thrombozytenfunktion in einer Vollblutprobe, wobei das Verfahren folgende Schritte umfasst:

a) Verwendung einer Messzelle, wobei die Messzelle folgende Elemente aufweist:

i) eine Haltekammer zum Halten der Probe,
ii) eine Kapillare, durch die die Probe von der Haltekammer in eine Messkammer geleitet wird,
iii) eine Messkammer, die durch ein Trennelement in zwei Kompartimente unterteilt ist, wobei das erste Kompartiment die Probe aus der Kapillare aufnimmt,
iv) ein Trennelement, das die Messkammer in zwei Kompartimente unterteilt und das eine Öffnung aufweist, durch welche die Probe aus dem ersten Kompartiment in das zweite Kompartiment fließen kann;

b) Befüllen der Haltekammer der Messzelle mit der Probe;
c) Platzieren der Messzelle in einem Gerät zur automatischen Bestimmung der Thrombozytenfunktion, wobei das Gerät folgende Elemente aufweist:

i) Mittel zum Anlegen eines Unterdrucks in der Messkammer der Messzelle,
ii) Mittel zur Bestimmung der Flussrate der Probe, die erzeugt wird, wenn in der Messkammer der Messzelle ein Unterdruck angelegt ist;

d) Anlegen eines Unterdrucks in der Messkammer der Messzelle und Durchleiten der Probe durch die Kapillare und durch die Öffnung des Trennelements;
e) Bestimmen der Flussrate der Probe, welche durch das Anlegen eines Unterdrucks durch die Kapillare und durch die Öffnung des Trennelements gezogen wird;
f) Bestimmen einer initialen Flussrate IF;
g) Bestimmen der Verschlusszeit CT, wobei der Zeitpunkt, in dem die Flussrate für eine Dauer von drei Sekunden weniger als 10 % der initialen Flussrate IF betragen hat, den Endpunkt der Verschlusszeit darstellt;
**dadurch gekennzeichnet, dass**
die Thrombozytenfunktion (PHC) nach folgender Formel bestimmt wird:

$$ \mathrm{PHC} = \frac{100 - FF\ [\%\ \mathrm{von}\ IF]}{(CT - CT_M) + 90} $$

mit
FF = finale Flussrate,
IF = initiale Flussrate,
CT = Verschlusszeit der Probe [s],
$CT_M$ = Referenzverschlusszeit [s] für normale Thrombozytenfunktion,
und wobei -wenn über eine Testdauer von mindestens 300 Sekunden keine finale Flussrate gemessen werden konnte, die gegenüber der initialen Flussrate um 90 % vermindert ist und dadurch keine Verschlusszeit bestimmt werden kann- für die Verschlusszeit CT 300 Sekunden festgelegt werden und die tatsächliche Flussrate zu diesem Zeitpunkt als finale Flussrate bestimmt wird.

**2.** Verfahren nach Anspruch 1, wobei die Thrombozytenfunktion in % der Norm bestimmt wird, indem der PHC-Wert mit 100 multipliziert wird.

**3.** Verfahren zur Langzeitkontrolle der Thrombozytenfunktion in einem Patienten, wobei Proben des Patienten zu unterschiedlichen Zeitpunkten bereitgestellt werden und jeweils die Thrombozytenfunktion mit einem Verfahren gemäß einem der vorhergehenden Ansprüche bestimmt wird.

**Claims**

**1.** Method for determining thrombocyte function in a whole blood sample, wherein the method comprises the following steps:

a) using a measuring cell, wherein the measuring cell comprises the following elements:

i) a retention chamber for retaining the sample,

ii) a capillary through which the sample is conducted from the retention chamber into a measuring chamber,

iii) a measuring chamber which is divided by a partition element into two compartments, wherein the first compartment accommodates the sample from the capillary,

iv) a partition element which divides the measuring chamber into two compartments and which has an aperture through which the sample can flow from the first compartment into the second compartment;

b) filling the retention chamber of the measuring cell with the sample;

c) placing the measuring cell in a device for automatic determination of thrombocyte function, wherein the device comprises the following elements:

i) means for applying negative pressure in the measuring chamber of the measuring cell,

ii) means for determining the flow rate of the sample, which is generated when negative pressure is applied in the measuring chamber of the measuring cell;

d) applying negative pressure in the measuring chamber of the measuring cell and conducting the sample through the capillary and through the aperture in the partition element;

e) determining the flow rate of the sample, which is drawn through the capillary and through the aperture in the partition element as a result of the application of negative pressure;

f) determining an initial flow rate IF;

g) determining the closure time CT, the end point of the closure time being the time at which the flow rate was less than 10% of the initial flow rate IF for a period of three seconds;

**characterized in that**

the thrombocyte function (PHC) is determined according to the following formula:

$$\text{PHC} = \frac{100 - FF\ [\%\ of\ IF]}{(CT - CT_M) + 90}$$

where

FF = final flow rate,

IF = initial flow rate,

CT = closure time of the sample [s],

$CT_M$ = reference closure time [s] for normal thrombocyte function,

and where - if, over a test period of at least 300 seconds, it was not possible to measure a final flow rate reduced by 90% with respect to the initial flow rate and it is thereby not possible to determine a closure time - 300 seconds are assigned to the closure time CT and the actual flow rate at this time is determined as the final flow rate.

2. Method according to Claim 1, wherein the thrombocyte function in % of the norm is determined by the PHC value being multiplied by 100.

3. Method for long-term monitoring of thrombocyte function in a patient, wherein samples from the patient are provided at different times and the thrombocyte function is determined in each case using a method according to either of the preceding claims.

**Revendications**

1. Procédé de détermination de la fonction des thrombocytes dans un échantillon de sang total, le procédé comprenant les stades suivants :

a) utilisation d'une cellule de mesure, la cellule de mesure ayant les éléments suivants :

i) une chambre de retenue pour retenir l'échantillon,

ii) un tube capillaire, par lequel l'échantillon va de la chambre de retenue à une chambre de mesure,

iii) une chambre de mesure, qui est subdivisée par un élément de cloisonnement en deux compartiments, le premier compartiment recevant l'échantillon du tube capillaire,

iv) un élément de cloisonnement, qui subdivise la chambre de mesure en deux compartiments et qui a une ouverture, par laquelle l'échantillon peut passer du premier compartiment au deuxième compartiment ;

b) le remplissage de la chambre de retenue de la cellule de mesure par l'échantillon ;
c) le placement de la cellule de mesure dans un appareil de détermination automatique de la fonction des thrombocytes, l'appareil ayant les éléments suivants :

i) des moyens d'application d'une dépression dans la chambre de mesure de la cellule de mesure,
ii) des moyens de détermination du débit de l'échantillon qui est produit, lorsqu'il est appliqué une dépression dans la chambre de mesure de la cellule de mesure ;

d) l'application d'une dépression dans la chambre de mesure de la cellule de mesure et le passage de l'échantillon dans le tube capillaire et dans l'ouverture de l'élément de cloisonnement ;
e) la détermination du débit de l'échantillon, qui, par l'application d'une dépression, passe par le tube capillaire et par l'ouverture de l'élément de cloisonnement ;
f) la détermination d'un débit IF initial ;
g) la détermination du temps CT de fermeture, l'instant où le débit a représenté pendant une durée de trois secondes moins de 10 % du débit IF initial représentant le point final du temps de fermeture ;
**caractérisé en ce que**
on détermine la fonction ( PHC ) des thrombocytes par la formule suivante :

$$PHC = \frac{100 - FF \text{ [\% de IF]}}{(CT - CT_M) + 90}$$

dans laquelle
FF = débit final,
IF = débit initial,
CT = temps de fermeture de l'échantillon [s],
$CT_M$ = temps de fermeture de référence [s] pour une fonction des thrombocytes normale,
et dans lequel si, pendant une durée de test d'au moins 300 secondes, on n'a pas pu mesurer de débit final, qui, par rapport au débit initial, est diminué de 90 %, et on n'a pas pu déterminer ainsi un temps de fermeture, on fixe 300 secondes pour le temps CT de fermeture et on détermine, comme débit final, le débit réel à cet instant.

**2.** Procédé suivant la revendication 1, dans lequel on détermine la fonction des thrombocytes en pourcentage de la norme, la valeur de PHC étant multipliée par 100.

**3.** Procédé de contrôle de longue durée de la fonction des thrombocytes chez un patient, dans lequel on se procure des échantillons du patient à des instants différents et on détermine respectivement la fonction des thrombocytes par un procédé suivant l'une des revendications précédentes.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9734698 A
- EP 1850134 A1